# EUROPÄISCHE PATENTANMELDUNG

(11) **EP 1 092 400 A2**
(43) Veröffentlichungstag der Anmeldung: **18.04.2001**
(21) Anmeldenummer: 00120055.9
(22) Anmeldetag: 15.09.2000
(51) Int. Cl.: A61F 2/06

(54) **Dickdarm-Stent**

(30) Priorität: 13.10.1999 DE 19949334
(71) Anmelder: Jostra Medizintechnik AG, 72145 Hirrlingen (DE)
(72) Erfinder: Breucha, Georg, 72379 Hechingen (DE); Sunnanväder, Lars, 72379 Hechingen (DE); Stumpp, Gerd, 72145 Hirrlingen (DE)
(74) Vertreter: Heuckeroth, Volker

(57) **Zusammenfassung**

Ein Stent (15) mit einem radial expandierbaren und in Längsrichtung (17) offenen rohrartigen Hohlkörper (16) ist zur Implantation in ein Hohlorgan des menschlichen Körpers, insbesondere in den Darm, vor allem in Rectum, Sigma, Colon descendens oder Colon transversum vorgesehen. Er weist Mittel (21) zur vorzugsweise lösbaren Fixierung des Stents (15) innen in dem Hohlorgan auf.

## Beschreibung

Die vorliegende Erfindung betrifft einen Stent mit einem radial expandierbaren und in Längsrichtung offenen rohrartigen Hohlkörper zur Implantation in ein Hohlorgan des menschlichen Körpers, insbesondere in den Darm, vor allem in Rectum, Sigma, Colon descendens oder Colon transversum.

Als Stents werden Endoprothesen bezeichnet, die bei der Behandlung von Stenosen in Hohlorganen vielfach etabliert sind. Derartige Stents werden bei pathologischen Verengungen oder äußerem Druck durch Tumore in Hohlorgane eingesetzt, um deren Lumen offenzuhalten. Als Einsatzbereich gelten bisher Blutgefäße, Speiseröhre, Gallengang und Harnwege.

Bei Patienten mit Speiseröhrenkrebs führt z.B. der Tumor in der Speiseröhrenwand zu Verengungen, die die Nahrungspassage in den Magen beeinträchtigen bis gänzlich unmöglich machen. Damit Patienten die Schluckfähigkeit und weitgehend normale Ernährung erhalten bleibt, wird häufig ein Stent in die Speiseröhre implantiert, der die Speiseröhre im Bereich des Tumors für den Durchtritt von Nahrung offenhält. Die Fixierung des Stents erfolgt durch Verkeilen des Stents im Tumorgewebe.

Ein aus der EP 0 878 174 A2 bekannter Stent ist für die Implantation in koronare Gefäße vorgesehen und dient dazu, diese aufzuweiten und in dem aufgeweiteten Zustand zu halten. Der Stent wird zu diesem Zweck in einem nicht-expandierten Zustand im Körper des Patienten positioniert und nachfolgend beispielsweise durch einen Ballonkatheter expandiert. Dies ist möglich, weil der Stent als rohrförmiger Körper aus Metall vorliegt, dessen Wand eine Stegstruktur aufweist, die mehrere zueinander benachbarte, durch Stege begrenzte Zellen aufweist. Durch Verformung der einzelnen Stegbereiche kann der Stent bei der Expansion auf einen wesentlich größeren Außendurchmesser aufgeweitet werden.

Bei Stents in Hohlorganen des Gastrointestinaltraktes kommt es dabei vor, daß diese bei ungenügender Verankerung durch den Tumor spontan abgehen, was einen Nachteil bekannter Stents darstellt.

Ein Einsatz von Stents im Dickdarm wurde bisher nicht in Betracht gezogen, weil der Dickdarm stärker mit Keimen besiedelt ist als z.B. die Speiseröhre, so daß eine endoluminale Druckbelastung der Darmwand zu Durchblutungsstörungen der Schleimhaut mit konsekutiver Infektion führen kann. Erschwerend kommt hinzu, daß der Dickdarm gewunden und flexibel fixiert ist und bei einer Länge von über 150 cm Stenosen aufweist, die einen flexiblen Applikator erforderlich machen, der andere Voraussetzungen als ein Applikator für Speiseröhren-Stents erfüllen muß.

Im Zusammenhang mit Dickdarmoperationen und chirurgischen Anastomosen kann es jedoch sowohl bei handgenähten als auch bei geklammerten Darmnähten zu einer konsekutiven narbigen Verengung der Darmlichtung kommen, die sich klinisch durch eine Passagestörung des Darminhaltes mit entsprechenden chronischen Beschwerden auswirken kann. Bei derartigen Beschwerden ist derzeit nur die neuerliche operative Nachresektion entsprechender narbiger Verengungen unter neuerlicher Darmnaht möglich. Als alternativ beschrittener Behandlungsweg wird mit einem transanal eingeführten Ballondilatator eine intraluminale Dilatation der Stenose unter Durchleuchtung durchgeführt. Bei Patienten mit stenosierenden Tumoren und schlechter Prognose bzw. bei Inoperabilität wegen schlechtem Allgemeinzustand besteht derzeit die Möglichkeit, als Operationsersatz entweder eine Colondilatation bzw. eine transanale transluminale Tumorverkleinerung mittels Laser durchzuführen.

Diese Verfahren erreichen jedoch häufig nicht zufriedenstellende Ergebnisse, da es bei gutartigen Stenosen durch narbige Schrumpfung erneut zu Restenosen kommen kann, die eine häufige Wiederholung der Dilatationsbehandlung erforderlich machen.

Auch bösartige Tumore führen durch ihr weiteres Wachstum zu neuerlichen Stenosen, so daß auch hier mehrfach Laserbehandlungen erforderlich sind. Neben einer übermäßigen Beeinträchtigung der Patienten stellen die wiederholten Behandlungen einen zusätzlichen Kostenfaktor dar, der in zunehmendem Maße Berücksichtigung findet.

Vor diesem Hintergrund liegt der vorliegenden Erfindung die Aufgabe zugrunde, den eingangs erwähnten Stent derart weiterzubilden, daß er bei einfacher Herstellungsmöglichkeit ein weiteres Anwendungsfeld findet.

Bei dem eingangs erwähnten Stent wird diese Aufgabe gelöst durch Mittel zur vorzugsweise lösbaren Fixierung des Stents innen in dem Hohlorgan.

Die der Erfindung zugrunde liegende Aufgabe wird auf diese Weise vollkommen gelöst.

Der Erfinder der vorliegenden Anmeldung hat nämlich erkannt, daß bisher gegen den Einsatz von Stents im Dickdarmbereich sprach, daß die Stents die Dickdarminnenwand nicht verletzen dürfen, um nicht die Gefahr von Ulceracation, Entzündungen und Abszessen mit Perforationsgefahr einzugehen. Ein weiteres Problem liegt darin, daß Fremdkörper zur Ausbildung von hypertrophen Pseudopolypen führen können.

Diese Probleme können jetzt weitgehend dadurch beseitigt werden, daß die Möglichkeit einer späteren Entfernung der neuen Stents gegeben ist, so daß sich für den neuen Stent ein weiterer Einsatzbereich erschließt, nämlich der im Dickdarmbereich.

Natürlich ist der neue Stent auch in andere Hohlorgane einsetzbar, wo die Entfernbarkeit eine untergeordnete oder keine Rolle spielt, die neue Art der Fixierung jedoch wichtig ist.

Dabei ist es bevorzugt, wenn der Hohlkörper eine Länge von höchstens ca. 15 cm, vorzugsweise ca. 10 cm, und im expandierten Zustand einen minimalen Lumendurchmesser von mindestens ca. 10 mm, vorzugsweise ca. 12-26 mm, aufweist.

Der Erfinder der vorliegenden Anmeldung hat erkannt, daß diese von Speiseröhren-Stents nicht erfüllten Abmaße ideal an die Bedingungen im Dickdarmbereich angepaßt sind.

Dabei ist es bevorzugt, wenn der Hohlkörper mit einer derartigen äußeren Beschichung, vorzugsweise aus Silikon, versehen ist, die ein Eindringen von Gewebe, vorzugsweise von Schleimhaut, in den Hohlkörper verhindert, wobei der Stent weiter vorzugsweise auf seiner äußeren Umfangsfläche als Mittel zur lösbaren Fixierung Vorsprünge aufweist, die beim Expandieren mit dem Hohlkörper innen in Anlage gelangen.

Der Vorteil dieser Maßnahmen ist vor dem Hintergrund zu sehen, daß eines der Grundprobleme bei Stents im Dickdarm die Schleimhautsituation darstellt, die anders als beim Plattenepithel der Speiseröhre und dem Zylinderepithel der Gallenwege Schleim sezerniert. Der Sekret- und Schleimbildung wird nun bei dem Aufbau des neuen Stents Rechnung getragen, indem durch die Vorsprünge ein Sekretabfluß gewährleistet wird. Zusätzlich bzw. alternativ wird durch die glatte Außenwand infolge der Beschichtung sichergestellt, daß der Schleimhaut oder einem Tumor das Eindringen in die Lichtung des Stents verwehrt wird, um Reizzustände zu verhindern und die Entfernung des Stents zu ermöglichen.

Die Vorsprünge, die gleichzeitig für eine lösbare Fixierung sorgen, können elastische Noppen, vorzugsweise aus Silikon, umfassen, wobei ferner vorzugsweise die Vorsprünge elastische Ringe oder Ringsegmente, ebenfalls vorzugsweise aus Silikon, umfassen.

Weiter ist es bevorzugt, wenn der Stent als Mittel zur lösbaren Fixierung auf seiner äußeren Umfangsfläche zumindest im expandierten Zustand eine etwa quer zur Längsrichtung umlaufende Vertiefung aufweist, die vorzugsweise in Längsrichtung etwa mittig angeordnet ist, wobei die Vertiefung weiter vorzugsweise zumindest teilweise in der äußeren Beschichtung vorgesehen ist und der Stent ferner vorzugsweise die Vertiefung auch im noch nicht-expandierten Zustand aufweist.

Durch diese Maßnahmen wird eine ungewollte Dislokation des Stents vermieden. Verglichen mit den Abstandshaltern handelt es sich bei der sozusagen umlaufenden Nut um eine alternative oder zusätzliche Möglichkeit der Fixierung, wobei die Noppen, Ringe oder Ringsegmente, die innen an der Darmwand anliegen, für einen Spalt zu dem Stent selbst sorgen, so daß der bereits erwähnte Sekretabfluß gewährleistet ist.

Alternativ kann es auch sinnvoll sein, keine umlaufende Nut oder Kehle sondern eine umlaufende Verdickung vorzusehen, die einen entsprechenden Druck auf die Naht ausübt.

Die Ausbildung der Vertiefung bzw. der Verdickung läßt sich durch entsprechende Einstellung der in Längsrichtung gesehenen Elastizität und Dehnbarkeit der Wand des Hohlkörpers erreichen, so daß sich beim Expandieren automatisch die Verstärkung /Verdickung ausbildet oder verstärkt. Diese Maßnahme ist technisch besonders bevorzugt, denn sie ermöglicht eine kostengünstige Herstellung der neuen Stents z.B. durch lasergeschnittene Stege und Zellen, deren Struktur in Längsrichtung derart unterschiedlich ist, daß sich der Stent abschnittsweise unterschiedlich stark ausdehnt, so daß hierdurch die Verdickung bzw. Vertiefung entsteht. Zusätzlich bzw. alternativ kann die Vertiefung/Verdickung auch zumindest teilweise in der äußeren Beschichtung vorgesehen sein.

Wenn der Stent im Bereich seiner offenen Enden wulstartige Verdickungen aufweist, so wird eine Beschädigung der Darminnenwand vermieden.

Wenn die Abstandshalter elastisch, vorzugsweise aus Silikon ausgebildet sind, läßt sich der Stent nach einer gewissen Zeit aus dem Darm entfernen. Wird alternativ/zusätzlich die umlaufende Nut eingesetzt, so erfolgt ggf. ein spontaner Abgang, wenn die Stenose ausreichend geweitet und die Darmwand elastischer geworden ist.

Der Einsatz eines Dickdarm-Stents ist für zwei verschiedene Indikationen gedacht:

Das erste Einsatzgebiet sind stenosierende Dickdarmtumore mit drohendem Darmverschluß bei Patienten, die entweder eine infauste Prognose haben oder wegen ihres schlechten Allgemeinzustandes inoperabel sind. Die Stentapplikation erspart eine Operation und bewahrt unter Erhalt der Darmlichtung vor einem Darmverschluß. Hier ist die dauerhafte Applikation des Stents sinnvoll.

Das zweite Einsatzgebiet der neuen Stents stellen narbige bzw. entzündliche Stenosen (Engstellungen) des Dickdarms nach operativen Eingriffen mit Dickdarmresektionen und Darmanastomosen bzw. entzündlichen Dickdarmerkrankungen (Divertikulitis) dar. Hier ist es sinnvoll, die narbige Verengung des Dickdarms mit einem Dilatator in mehreren Sitzungen aufzuweiten, um anschließend mit einem als Applikator ausgebildeten Dilatator den Stent als Platzhalter in der Stenose zu verankern. Dies ist insbesondere deshalb von Vorteil, weil narbige Stenosen auch nach mehrfacher Dilatation die Tendenz zur neuerlichen Schrumpfung und Einengung haben, dem durch die Applikation des neuen Stents entgegengewirkt wird. Um das genaue Plazieren des Stents in der Stenose zu gewährleisten, ist dabei der Applikator mit einem Röntgenkontraststreifen versehen.

Neben der geplanten Entfernung eines Stents, z.B. aus Metall stellen spontane Abgänge nach Relaxation der Stenosenwand bzw. die Autolyse von Stents aus resorbierbarem Material alternative Möglichkeiten dar.

Weitere Vorteile ergeben sich aus der Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung ist in der Zeichnung dargestellt und wird in der nachfolgenden Beschreibung näher erläutert. Es zeigen:
- Fig. 1: einen schematischen Längsschnitt durch den Darm mit einer Darmnaht, bei der die Schleimhaut eingestülpt ist;
- Fig. 2: einen Stent mit umlaufender Nut in der Außenwand und Wülsten an den Enden;
- Fig. 3: einen Stent mit umlaufender Verdickung und radial verteilten Vorsprüngen; und
- Fig. 4: einen Stent mit umlaufender, flacher Vertiefung in der Beschichtung und radial verteilten Vorsprüngen.

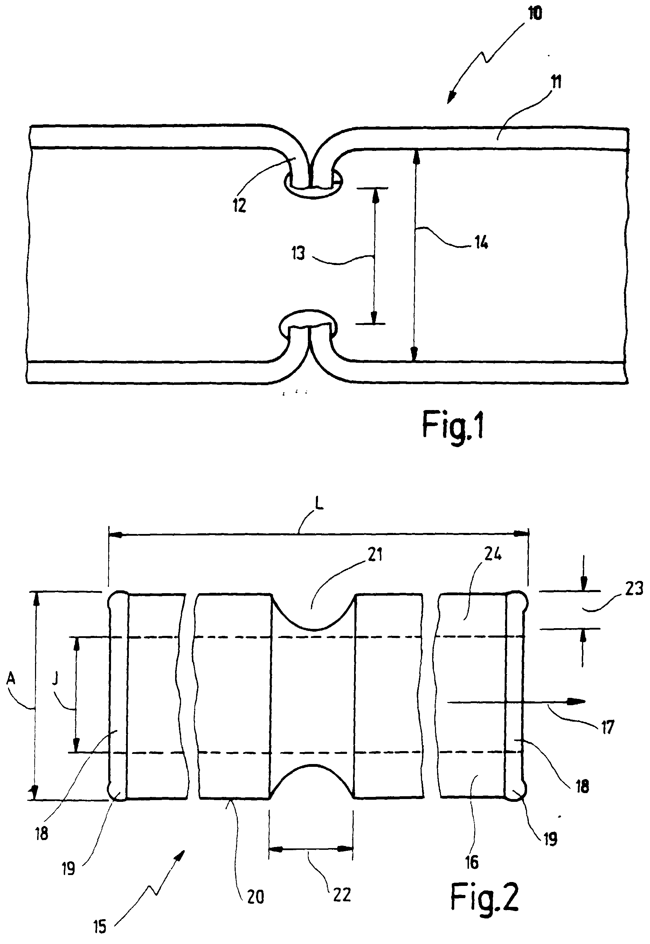

In Fig. 1 ist schematisch ein Darm 10 im Längsschnitt dargestellt, dessen Darmwand 11 zur Anlegung einer Darmnaht 12 nach innen gestülpt ist. Im Bereich der Darmnaht 12 ergibt sich eine Verengung 13 des ursprünglich größeren Lumens 14 des Darmes 10, wodurch eine Passagestörung des Darminhaltes mit entsprechenden chronischen Beschwerden hervorgerufen werden kann.

Um einen drohenden Darmverschluß nach einem operativen Eingriff oder alternativ zu einem operativen Eingriff zu verhindern, wird eine als Stent bezeichnete Endoprothese aus medizinischem Stahl bzw. resorbierbarem Material eingesetzt, die in Fig. 2 in schematischer Seitenansicht gezeigt ist. Dieser Stent 15 ist ein rohrartiger Hohlkörper 16, der in Längsrichtung 17 offen ist. Er weist eine bevorzugte Länge L von 10 cm, einen bevorzugten Außendurchmesser A von 28-31 mm und einen bevorzugten Innendurchmesser I von 10-26 mm auf. An seinen offenen Enden 18 weist der Stent 15 wulstartige Verdickungen 19 auf, durch die eine Beschädigung der Darminnenwand vermieden wird.

In seiner äußeren Umfangsfläche 20 ist eine umlaufende Vertiefung 21 vorgesehen, deren Breite 22 ca. 10 mm und deren Tiefe 23 ca. 3-4 mm beträgt, so daß der Stent 15 in seiner Wand 24 eine Art Kehle aufweist, die von den Abmaßen her an die Darmnaht 12 angepaßt ist.

Beim Einsetzen des Stent 15 in den Darm 10 wird dieser so zu der Darmnaht 12 positioniert, daß die umlaufende Nut 21 beim Expandieren des Stents 15 in radialer Richtung für eine Fixierung des Stents 15 an der Darmnaht 12 sorgt.

Über die Nut 21 wird insofern für eine lösbare Fixierung des Stents 15 in dem Darm 10 gesorgt, als der Stent wieder entfernbar ist, wenn die Verengung 13 stärker geweitet wurde und/oder die Darmwand 11 im Bereich der Darmnaht 12 elastischer geworden ist. Der Stent 15 kann dabei entweder geplant entfernt werden oder aber spontan abgehen.

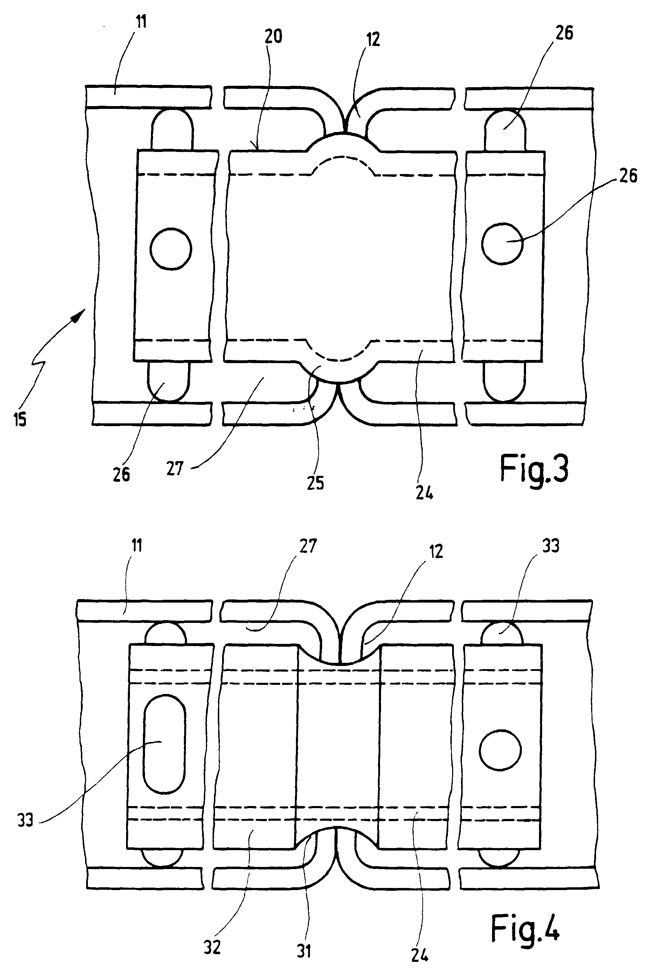

Anstelle der Vertiefung 21 kann es auch sinnvoll sein, an der äußeren Umfangsfläche 20 des Stents 15 eine umlaufende Verdickung 25 vorzusehen, wie dies in Fig. 3 gezeigt ist. Zusätzlich weist der Stent 15 aus Fig. 3 auf seiner äußeren Umfangsfläche 20 noch elastische Vorsprünge auf, die vorzugsweise aus Silikon gefertigt sind. Die Vorsprünge können elastische Noppen 26 oder aber elastische Ringe oder Ringsegmente sein. Sie sorgen dafür, daß zwischen der Darmwand 11 und dem Stent 15 ein Spalt 27 für Sekretabfluß verbleibt.

Um ein Eindringen von Tumorgewebe oder Schleimhaut in den Stent 15 zu verhindern, weist dieser eine glatte Außenhaut auf, die - wie in Fig. 4 dargestellt - durch eine Beschichtung 32 vorzugsweise aus Silikon realisiert werden kann. Alternativ ist es auch möglich, die äußere Umfangsfläche unmittelbar glatt auszubilden.

In Fig. 4 ist zu erkennen, daß auch dort eine umlaufende Vertiefung 31 vorgesehen ist, die für eine vorübergehende Fixierung des Stents 15 an der Darmnaht 12 sorgt. Auf der Beschichtung 32 sind in Fig. 4 Ringsegmente 33 vorgesehen, die als Abstandshalter wirken und den bereits aus Fig. 3 bekannten Spalt 27 zur Darmwand 12 für Sekretabfluß freilassen.

Der neue Stent 15 weist die Vertiefung 21, 31 bzw. Verdickung 25 einerseits in seinem in den Figuren 2 bis 4 gezeigten expandierten Zustand auf, was z.B. dadurch erreicht werden kann, daß die Elastizität und Dehnbarkeit des Stents in Längsrichtung 17 derart unterschiedlich ist, daß sich Vertiefung 21, 31 bzw. Verdickung 25 erst beim Expandieren ausbilden. Dies wird dadurch erreicht, daß die Stege und Zellen, die bei der Fertigung des Stents entstehen, eine entsprechend unterschiedliche Struktur haben.

Andererseits ist es auch möglich, vor der Fertigung des Stents dessen Wand 24 durch chemische oder mechanische Verfahren derart vorzubearbeiten, daß eine umlaufende Vertiefung 21, 31 bzw. Verdickung 25 entsteht, die beim Expandieren erhalten bleibt.

## Patentansprüche

1. Stent mit einem radial expandierbaren und in Längsrichtung offenen rohrartigen Hohlkörper (16) zur Implantation in ein Hohlorgan (10) des menschlichen Körpers, insbesondere in den Darm, vor allem in Rectum, Sigma, Colon descendens oder Colon transversum, gekennzeichnet durch Mittel (21, 25, 26, 31, 33) zur vorzugsweise lösbaren Fixierung des Stents (15) innen in dem Hohlorgan (10).

2. Stent nach Anspruch 1, dadurch gekennzeichnet, daß der Hohlkörper (16) eine Länge von höchstens ca. 15 cm, vorzugsweise ca. 10 cm und im expandierten Zustand einen minimalen Lumendurchmesser (I) von mindestens ca. 10 mm, vorzugsweise ca. 12-26 mm aufweist.

3. Stent nach Anspruch 1 oder 2, dadurch gekennzeichnet, daß der Hohlkörper (16) mit einer derartigen äußeren Beschichtung (32), vorzugsweise aus Silikon, versehen ist, die ein Eindringen von Gewebe, vorzugsweise von Schleimhaut, in den Hohlkörper (16) verhindert.

4. Stent nach einem der Ansprüche 1 bis 3, dadurch gekennzeichnet, daß er auf seiner äußeren Umfangsfläche (20) als Mittel (21, 25, 26, 31, 33) zur lösbaren Fixierung Vorsprünge (26, 33) aufweist, die beim Expandieren mit dem Hohlkörper (10) innen in Anlage gelangen.

5. Stent nach Anspruch 4, dadurch gekennzeichnet, daß die Vorsprünge (26, 33) elastische Noppen (26), vorzugsweise aus Silikon umfassen.

6. Stent nach Anspruch 4 oder 5, dadurch gekennzeichnet, daß die Vorsprünge (26, 33) elastische Ringe oder Ringsegmente (33), vorzugsweise aus Silikon umfassen.

7. Stent nach einem der Ansprüche 1 bis 6, dadurch gekennzeichnet, daß er als Mittel (21, 25, 26, 31, 33) zur lösbaren Fixierung auf seiner äußeren Umfangsfläche (20) zumindest im expandierten Zustand eine etwa quer zur Längsrichtung (17) umlaufende Vertiefung (21, 31) aufweist, die vorzugsweise in Längsrichtung (17) etwa mittig angeordnet ist.

8. Stent nach Anspruch 3 und 7, dadurch gekennzeichnet, daß die Vertiefung (31) zumindest teilweise in der äußeren Beschichtung (32) vorgesehen ist.

9. Stent nach Anspruch 7, dadurch gekennzeichnet, daß er die Vertiefung (21, 31) auch im noch nicht-expandierten Zustand aufweist.

10. Stent nach einem der Ansprüche 7 bis 9, dadurch gekennzeichnet, daß der Hohlkörper (16) eine Wand (24) aufweist, deren Elastizität und Dehnbarkeit in Längsrichtung (17) gesehen derart unterschiedlich ist, daß sie beim Expandieren die Vertiefung (21, 31) ausbildet oder verstärkt.

11. Stent nach einem der Ansprüche 1 bis 6, dadurch gekennzeichnet, daß der Hohlkörper (16) eine Wand (24) aufweist, deren Elastizität und Dehnbarkeit in Längsrichtung (17) gesehen derart unterschiedlich ist, daß sie beim Expandieren eine quer zur Längsrichtung umlaufende Verdickung (25) bildet oder verstärkt.

12. Stent nach einem der Ansprüche 1 bis 11, dadurch gekennzeichnet, daß er aus resorbierbarem Material gefertigt ist.

13. Stent nach einem der Ansprüche 1 bis 12, dadurch gekennzeichnet, daß er im Bereich seiner offenen Enden (19) wulstartige Verdickungen (20) aufweist.

14. Stent nach einem der Ansprüche 7 bis 10, dadurch gekennzeichnet, daß die Vertiefung (21) in Längsrichtung (17) eine maximale Breite (22) von ca. 10 mm sowie in radialer Richtung eine Tiefe (23) von ca. 3-4 mm aufweist.
